(19) Europäisches Patentamt
European Patent Office
Office européen des brevets

(11) EP 2 757 152 A1

(12) **EUROPEAN PATENT APPLICATION**
published in accordance with Art. 153(4) EPC

(43) Date of publication:
**23.07.2014 Bulletin 2014/30**

(21) Application number: **12831615.5**

(22) Date of filing: **29.08.2012**

(51) Int Cl.:
***C12N 15/00*** *(2006.01)* ***A61K 31/713*** *(2006.01)*
***A61K 48/00*** *(2006.01)* ***A61P 35/00*** *(2006.01)*
***C12N 15/09*** *(2006.01)* ***C12N 15/113*** *(2010.01)*
***C12Q 1/02*** *(2006.01)* ***C12Q 1/68*** *(2006.01)*
***G01N 33/15*** *(2006.01)* ***G01N 33/50*** *(2006.01)*

(86) International application number:
**PCT/JP2012/071868**

(87) International publication number:
**WO 2013/038907 (21.03.2013 Gazette 2013/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2011 JP 2011200756**

(71) Applicant: **Nippon Kayaku Kabushiki Kaisha**
**Tokyo 102-8172 (JP)**

(72) Inventor: **SATO, Takamichi**
**Tokyo 115-8588 (JP)**

(74) Representative: **Andrae | Westendorp**
**Patentanwälte Partnerschaft**
**Uhlandstraße 2**
**80336 München (DE)**

(54) **METHOD FOR INHIBITING CELL GROWTH, NUCLEIC ACID MOLECULE HAVING RNA INTERFERENCE EFFECT ON NEK10 VARIANT GENE, AND ANTICANCER AGENT**

(57) The present invention provides a method for inhibiting cell growth, a nucleic acid molecule useful as an anticancer agent, and a method for screening novel anticancer agents. In the present invention, inhibitory effects on expression of NEK10 variant gene or inhibitory effects on activity of NEK10 variant protein are obtained in cells by transfecting cells with a nucleic acid molecule having an RNA interference effect on NEK10 variant gene. The present invention also provides a method for screening anticancer agents by using this inhibitory effect as an indicator.

EP 2 757 152 A1

## Description

### TECHNICAL FIELD

The present invention relates to a method for inhibiting cell growth, a nucleic acid molecule having an RNA interference effect on NEK10 variant gene, a composition for inhibiting NEK10 variant gene expression comprising an expression vector for expressing that nucleic acid molecule in cells and that nucleic acid molecule, an anticancer agent having that nucleic acid molecule has an active ingredient thereof, and a method for screening anticancer agents.

### BACKGROUND ART

Cancer is the leading cause of death in modern-day Japan, with more than 30,000 people dying from cancer annually. Despite advances having been made in the detection and treatment of cancer, mortality rates associated with cancer remain high. Although molecular-targeted anticancer agents such as Glivec™ and Herceptin™ have recently attracted attention for use in cancer chemotherapy, since patients in whom they have demonstrated efficacy are limited, there is a desire for the development of a novel molecular-targeted anticancer agent.

Starting in the first part of the 1970's, NIMA kinase was discovered in Aspergillus nidulans, and a homologue of NIMA kinase in the form of Fin1 was discovered in fission yeast. NIMA kinase belongs to the serine/threonine kinase family, and has been indicated to play an important role during the M phase of the cell cycle. Namely, NIMA kinase was clearly determined to be a molecule that plays a central role in entering into the M phase, control of chromosome condensation, spindle formation and cytoplasmic division (see, for example, Non-Patent Document 1).

Homologues of human NIMA kinase consist of NIMA-related kinases (NEK) in the form of NEK1 to NEK11, and these kinases constitute the NEK family. Until now, the NEK family was known to consist of important molecules in the cell cycle and signaling pathways. For example, the NEK family members of NEK2, NEK6, NEK7 and NEK9 have been reported to be involved in entering into the M phase, control of chromosome condensation, spindle formation and cytoplasmic division in the same manner as NIMA kinase and Fin1 (see, for example, Non-Patent Document 2).

NEK10 has been reported to play an important role in controlling the G2/M phase checkpoint in response to UV irradiation (see, for example, Non-Patent Document 3). An SNP present in NEK10 gene has been reported to be involved in the incidence of breast cancer (see, for example, Non-Patent Documents 4 and 5) . However, it is unclear as to whether NEK10 is involved in cell growth, and particularly the growth of cancer cells. In addition, a molecule presumed to be a human NEK10 variant is registered in the NCBI database (accession no. : AK098832.1, to be referred to as human NEK10 variant). Although there is the possibility that human NEK10 variant is also involved in control of the cell cycle since it has a kinase segment similar to that of other molecules of the NEK family, what types of functions it possesses in the body are unknown.

Prior Art Documents

Patent Documents

Non-Patent Document 1: M.J. O'Connell, et al., Trends in Cell Biology, 2003, Vol. 13, pp. 221-228
Non-Patent Document 2: L. O'Regan, et al., Cell Division, 2007, Vol. 2, pp. 25-36
Non-Patent Document 3: L.S. Moniz, et al., Molecular and Cellular Biology, 2011, Vol. 31, pp. 30-42
Non-Patent Document 4: S. Ahmed, et al., Nature Genetics, 2009, Vol. 41, pp. 585-590
Non-Patent Document 5: A.C. Antoniou, et al., Cancer Research, 2010, Vol. 70, pp. 9742-9754

### DISCLOSURE OF THE INVENTION

Problems to be Solved by the Invention

An object of the present invention is to provide a method for inhibiting cell growth, a nucleic acid molecule useful as an anticancer agent, and a method of screening novel anticancer agents.

Means for Solving the Problems

The inventor of the present invention thought that, since a molecule having homology with human NEK10 variant is present in mice and NEK10 variant is preserved across species, NEK10 variant may be important in cell growth and control of the cell cycle, and therefore conducted extensive research on NEK10 variant since its biological function is unknown. As a result, the inventor of the present invention found that cell growth is inhibited when expression of NEK10 variant is inhibited in cells, and that a nucleic acid having an RNA interference effect on NEK10 variant mRNA (NEK10 siRNA) has a cell growth inhibitory action on cells, and particularly on cancer cells, thereby leading to completion of the present invention.

Namely, the present invention adopts the constitutions indicated below.

(1) A method for inhibiting cell growth, comprising:

an expression decreasing step for decreasing expression of NEK10 variant gene, or
an activity decreasing step for decreasing activity of NEK10 variant protein
in cells.

(2) The method for inhibiting cell growth described in (1) above, wherein the expression decreasing step is a step for transfecting cells with at least one type selected from the group consisting of a nucleic acid molecule that inhibits expression of NEK10 variant gene by RNA interference, a precursor of the nucleic acid molecule, and an expression vector capable of expressing the nucleic acid molecule or the precursor.

(3) The method for inhibiting cell growth described in (2) above, wherein the nucleic acid molecule is siRNA having an RNA interference effect that targets a base sequence in mRNA of NEK10 variant gene represented by SEQ ID NO: 2 or SEQ ID NO: 4, and
the precursor is shRNA having an RNA interference effect that targets a base sequence in mRNA of NEK10 variant gene represented by SEQ ID NO: 2 or SEQ ID NO: 4.

(4) The method for inhibiting cell growth described in (2) or (3) above, wherein the nucleic acid molecule is selected from the group consisting of:

(a) siRNA consisting of the combination of sense RNA consisting of the base sequence represented by SEQ ID NO: 2 and antisense RNA consisting of the base sequence represented by SEQ ID NO: 3,
(b) siRNA consisting of the combination of sense RNA consisting of the base sequence represented by SEQ ID NO: 4, and antisense RNA consisting of the base sequence represented by SEQ ID NO: 5,
(c) siRNA consisting of the combination of sense RNA containing a contiguous base sequence of 15 to 24 bases in the base sequence represented by SEQ ID NO: 2, and antisense RNA containing a base sequence complementary to the sense RNA; and having an RNA interference effect on NEK10 variant gene,
(d) siRNA consisting of the combination of sense RNA containing a contiguous base sequence of 15 to 24 bases in the base sequence represented by SEQ ID NO: 4, and antisense RNA containing a base sequence complementary to the sense RNA; and having an RNA interference effect on NEK10 variant gene,
(e) siRNA consisting of the combination of sense RNA containing a base sequence in which one or a plurality of the bases in a contiguous base sequence of 15 or more bases in the base sequence represented by SEQ ID NO: 2 is substituted, added or deleted, and antisense RNA containing a base sequence complementary to the sense RNA; and having an RNA interference effect on NEK10 variant gene,
(f) siRNA consisting of the combination of sense RNA containing a base sequence in which one or a plurality of the bases in a contiguous base sequence of 15 or more bases in the base sequence represented by SEQ ID NO: 4 is substituted, added or deleted, and antisense RNA containing a base sequence complementary to the sense RNA; and having an RNA interference effect on NEK10 variant gene, and
(g) siRNA in which one or a plurality of bases in the siRNA described in any of (a) to (f) is modified, and having an RNA interference effect on NEK10 variant gene.

(5) The method for inhibiting cell growth described in any of (2) to (4) above, wherein the precursor produces in cells any of:

(a) siRNA consisting of the combination of sense RNA consisting of the base sequence represented by SEQ ID NO: 2 and antisense RNA consisting of the base sequence represented by SEQ ID NO: 3,
(b) siRNA consisting of the combination of sense RNA consisting of the base sequence represented by SEQ ID NO: 4 and antisense RNA consisting of the base sequence represented by SEQ ID NO: 5,
(c) siRNA consisting of the combination of sense RNA containing a contiguous base sequence of 15 to 24 bases in the base sequence represented by SEQ ID NO: 2, and antisense RNA containing a base sequence complementary to the sense RNA; and having an RNA interference effect on NEK10 variant gene,
(d) siRNA consisting of the combination of sense RNA containing a contiguous base sequence of 15 to 24 bases in the base sequence represented by SEQ ID NO: 4, and antisense RNA containing a base sequence complementary to the sense RNA; and having an RNA interference effect on NEK10 variant gene,

(e) siRNA consisting of the combination of sense RNA containing a base sequence in which one or a plurality of the bases in a contiguous base sequence of 15 or more bases in the base sequence represented by SEQ ID NO: 2 is substituted, added or deleted, and antisense RNA containing a base sequence complementary to the sense RNA; and having an RNA interference effect on NEK10 variant gene,

(f) siRNA consisting of the combination of sense RNA containing a base sequence in which one or a plurality of the bases in a contiguous base sequence of 15 or more bases in the base sequence represented by SEQ ID NO: 4 is substituted, added or deleted, and antisense RNA containing a base sequence complementary to the sense RNA; and having an RNA interference effect on NEK10 variant gene, or

(g) siRNA in which one or a plurality of bases in the siRNA described in any of (a) to (f) is modified, and having an RNA interference effect on NEK10 variant gene.

(6) The method for inhibiting cell growth described in (2) above, wherein the precursor is:

(p) shRNA containing

a contiguous base sequence of 15 or more bases in the base sequence represented by SEQ ID NO: 2 or a base sequence in which one or a plurality of bases in that base sequence is modified, substituted, added or deleted, and

a contiguous base sequence of 15 or more bases in the base sequence represented by SEQ ID NO: 3 or a base sequence in which one or a plurality of bases in that base sequence is modified, substituted, added or deleted,

or

(q) shRNA containing

a contiguous base sequence of 15 or more bases in the base sequence represented by SEQ ID NO: 4 or a base sequence in which one or a plurality of bases in that base sequence is modified, substituted, added or deleted, and

a contiguous base sequence of 15 or more bases in the base sequence represented by SEQ ID NO: 5 or a base sequence in which one or a plurality of bases in that base sequence is modified, substituted, added or deleted; and, siRNA, having an RNA interference effect on NEK10 variant gene, is produced in cells from the shRNA of (p) or the shRNA of (q) .

(7) The method for inhibiting cell growth described in any of (1) to (6) above, wherein the cells are cancer cells.

(8) A nucleic acid molecule that is:

(a) siRNA consisting of the combination of sense RNA consisting of the base sequence represented by SEQ ID NO: 2 and antisense RNA consisting of the base sequence represented by SEQ ID NO: 3,

(b) siRNA consisting of the combination of sense RNA consisting of the base sequence represented by SEQ ID NO: 4 and antisense RNA consisting of the base sequence represented by SEQ ID NO: 5,

(c) siRNA consisting of the combination of sense RNA containing a contiguous base sequence of 15 to 24 bases in the base sequence represented by SEQ ID NO: 2, and antisense RNA containing a base sequence complementary to the sense RNA; and having an RNA interference effect on NEK10 variant gene,

(d) siRNA consisting of the combination of sense RNA containing a contiguous base sequence of 15 to 24 bases in the base sequence represented by SEQ ID NO: 4, and antisense RNA containing a base sequence complementary to the sense RNA; and having an RNA interference effect on NEK10 variant gene,

(e) siRNA consisting of the combination of sense RNA containing a base sequence in which one or a plurality of the bases in a contiguous base sequence of 15 or more bases in the base sequence represented by SEQ ID NO: 2 is substituted, added or deleted, and antisense RNA containing a base sequence complementary to the sense RNA; and having an RNA interference effect on NEK10 variant gene,

(f) siRNA consisting of the combination of sense RNA containing a base sequence in which one or a plurality of the bases in a contiguous base sequence of 15 or more bases in the base sequence represented by SEQ ID NO: 4 is substituted, added or deleted, and antisense RNA containing a base sequence complementary to the sense RNA; and having an RNA interference effect on NEK10 variant gene, or

(g) siRNA in which one or a plurality of bases in the siRNA described in any of (a) to (f) is modified, and having an RNA interference effect on NEK10 variant gene.

(9) A nucleic acid molecule that is:

(p) shRNA containing

a contiguous base sequence of 15 or more bases in the base sequence represented by SEQ ID NO: 2 or a base sequence in which one or a plurality of bases in that base sequence is modified, substituted, added or deleted, and

a contiguous base sequence of 15 or more bases in the base sequence represented by SEQ ID NO: 3 or a base sequence in which one or a plurality of bases in that base sequence is modified, substituted, added or deleted,

or

(q) shRNA containing

a contiguous base sequence of 15 or more bases in the base sequence represented by SEQ ID NO: 4 or a base sequence in which one or a plurality of bases in that base sequence is modified, substituted, added or deleted, and

a contiguous base sequence of 15 or more bases in the base sequence represented by SEQ ID NO: 5 or a base sequence in which one or a plurality of bases in that base sequence is modified, substituted, added or deleted;

and,

is a precursor for producing siRNA having an RNA interference effect on NEK10 variant gene in cells.

(10) An expression vector containing the nucleic acid described in (8) or (9) above that is capable of expressing that nucleic acid.

(11) A composition for inhibiting expression of NEK10 variant gene, comprising one or more types selected from the group consisting of:

the nucleic acid molecule described in (8) above,
the nucleic acid molecule described in (9) above, and
the expression vector described in (10) above.

(12) An anticancer agent containing as an active ingredient thereof one or more types selected from the group consisting of:

the nucleic acid molecule described in (8) above,
the nucleic acid molecule described in (9) above, and
the expression vector described in (10) above.

(13) A method for screening anticancer agents using an inhibitory effect on expression of NEK10 variant gene or an inhibitory effect on activity of NEK10 variant protein as an indicator.

(14) The method for screening anticancer agents described in (13) above, comprising:

a step for culturing NEK10 variant-expressing cells respectively in the presence and absence of a candidate substance for an inhibitory effect on expression of NEK10 variant gene or an inhibitory effect on activity of NEK10 variant protein, and

a step for measuring the expression level of NEK10 variant mRNA in cells or the amount of activity of NEK10 variant protein, and comparing the expression levels or amounts of activity in the presence and absence of the candidate substance.

Effects of the Invention

According to the method for inhibiting cell growth of the present invention, cell growth can be inhibited by acting on a novel pathway that decreases activity of NEK10 variant gene. In addition, the nucleic acid molecule of the present invention is a substance that has an RNA interference effect on NEK10 variant mRNA, and is preferably used in the method for inhibiting cell growth of the present invention. Namely, the method for inhibiting cell growth and the nucleic acid molecule of the present invention are extremely useful in the treatment of cancer, and can be expected to demonstrate antitumor effects even in cancer patients for which effects were unable to be obtained with conventional growth inhibition methods.

In addition, according to the method for screening anticancer agents of the present invention, novel candidate compounds of anticancer agents can be acquired.

BRIEF DESCRIPTION OF THE DRAWINGS

FIG. 1 is a graph indicating relative NEK10 variant mRNA expression levels of each siRNA treatment group based on a value of 100 for the NEK10 variant mRNA expression level during treatment with control siRNA in Example 1.
FIG. 2 is a graph indicating cell growth rates of each siRNA treatment group based on a value of 100 for cell growth during treatment with control siRNA in Example 1.

BEST MODE FOR CARRYING OUT THE INVENTION

<Method for Inhibiting Cell Growth>

The method for inhibiting cell growth of the present invention is characterized as comprising an expression decreasing step for decreasing expression of NEK10 variant gene, and/or an activity decreasing step for decreasing the activity of NEK10 variant protein (protein encoded by NEK10 variant gene), in cells. Cell growth can be inhibited by inhibiting the function of NEK10 variant protein in cells. This is thought to be a phenomenon that occurs because NEK10 variant protein is involved in cell growth in cells. There are no particular limitations on the method used to decrease expression of NEK10 variant protein or decrease the activity of NEK10 variant protein, and any known technique used to decrease the expression of a specific gene or the activity of a protein in cells may be used.
An example of a method used to decrease the activity of NEK10 variant protein consists of transfecting cells with a substance that binds to NEK10 variant protein, such as antibody to NEK10 variant protein, and inhibiting the interaction of NEK10 variant protein with other biomolecules by causing that substance to bind with intracellular NEK10 variant protein. There are no particular limitations on the method used to transfect cells with a substance that binds to NEK10 variant protein, and the substance may be used to transfect the cells directly by injection and the like, or the substance may be allowed to contact the cell surface and transfect the cells by endocytosis and the like. In addition, NEK10 variant protein is predicted to have kinase activity in the same manner as NEK10 protein based on the structure of NEK10 variant protein. Thus, the activity of NEK10 variant protein can be inhibited by expressing within cells a dominant negative form in which the kinase active site of NEK10 variant protein has been deleted or substituted.
On the other hand, an example of a method for decreasing expression of NEK10 variant gene consists of knocking down NEK10 variant gene by RNA interference. More specifically, cells are transfected with a nucleic acid molecule that induces RNA interference targeted at mRNA of NEK10 variant gene (NEK10 variant mRNA) and causes degradation of NEK10 variant mRNA within the cells, such as an antisense nucleic acid, ribozyme nucleic acid or double-stranded RNA (dsRNA).
In general, RNA interference refers to a phenomenon by which expression of a target gene is inhibited by administering into cells small interfering RNA (siRNA) consisting of dsRNA formed from sense siRNA consisting of a partially homologous sequence of the mRNA sequence of the target gene and antisense siRNA consisting of a sequence complementary thereto, thereby causing the siRNA in the cells to separate into sense siRNA and antisense siRNA and allowing the target gene mRNA and the antisense siRNA to form a double strand, after which the formed dsRNA is degraded by Dicer. In addition to transfecting cells with siRNA, RNA interference can also be used to inhibit expression of a target gene in the same manner as siRNA by transfecting cells with a comparatively long-stranded dsRNA or hairpin shRNA (small hairpin RNA) serving as an siRNA precursor, or by transfecting with a vector that expresses siRNA or a precursor thereof. Moreover, methods are also known for inhibiting expression of a target gene by siRNA in vivo (P. Anton, et al., Nature, 2002, Vol. 418, pp. 38-39; L. David, et al., Nat. Genet., 2002, Vol. 32, pp. 107-108).

[Nucleic Acid Molecule having RNA Interference Effect on NEK10 Variant Gene]

In the present invention, expression of NEK10 variant gene is preferably decreased by transfecting cells with a nucleic acid molecule having an RNA interference effect on NEK10 variant gene (to also be referred to as the "RNAi nucleic acid molecule"). There are no particular limitations on the RNAi interference nucleic acid molecule provided it has an RNA interference effect on NEK10 variant mRNA, and is preferably siRNA.
The siRNA used in the present invention for the RNAi nucleic acid molecule may be siRNA for any region of NEK10 variant mRNA provided it has an RNA interference effect on that region. siRNA having an RNA interference effect on a particle base sequence of NEK10 variant mRNA can be prepared by a person with ordinary skill in the art by suitably designing based on base sequence information of NEK10 variant mRNA. For example, the base sequence of cDNA of human NEK10 variant mRNA is shown in SEQ ID NO: 1. Although the siRNA used in the present invention preferably has a base sequence that is totally complementary with the target base sequence in NEK10 variant mRNA, it may contain one or a plurality of mismatches provided it has an RNA interference effect.

There are no particular limitations on the base pair length of the siRNA used in the present invention provided it demonstrates an RNA interference effect on NEK10 variant mRNA. An example thereof is siRNA in which sense RNA and antisense RNA consist of 15 to 30 base pairs and preferably 21 to 23 base pairs.

In addition, the siRNA used in the present invention may have one or a plurality of modified bases provided it has an RNA interference effect. Examples of these modifications include methylation, inosinylation, dU formation, fluorescent group modification and phosphorylation. The modified base may be present in siRNA in sense RNA, antisense RNA or both.

The end structure of the siRNA used in the present invention may be that of a blunt end or an overhanging end provided it allows expression of NEK10 variant gene to be regulated by an RNA interference effect. An overhanging end structure can include not only a structure in which the 3'-end protrudes, but also a structure in which the 5'-end that demonstrates the aforementioned RNA interference effect protrudes. In addition, although the number of overhanging bases is 2 to 3 in numerous siRNA previously reported to demonstrate an RNA interference effect on other genes, the number of bases, in the siRNA used in the present invention, is only required to be that capable of inducing an RNA interference effect, and for example, the number of overhanging bases may be 1 to 8 and preferably 2 to 4. These overhanging bases are not required to constitute a sequence complementary (antisense) or identical (sense) to NEK10 variant mRNA.

Examples of siRNA having an RNA interference effect on NEK10 variant mRNA include siRNA having an RNA interference effect targeting a contiguous base sequence of the entire or partial length of the base sequence represented by SEQ ID NO: 2 in NEK10 variant mRNA, such as a contiguous base sequence of 15 to 24 bases and preferably a contiguous base sequence of 18 to 20 bases. Another example is siRNA having an RNA interference effect targeting a contiguous base sequence of the entire or partial length of the base sequence represented by SEQ ID NO. : 4 in NEK10 variant mRNA, such as a contiguous base sequence of 15 to 24 bases and preferably a contiguous base sequence of 18 to 20 bases.

Specific examples of siRNA targeting the entire length, or a partial length thereof, of the base sequence represented by SEQ ID NO: 2 in NEK10 variant mRNA include: siRNA consisting of the combination of sense RNA consisting of the base sequence represented by SEQ ID NO: 2 (5'-GAAAUCCUGUCAGAUGAUAACUUCA-3') and antisense RNA consisting of the base sequence represented by SEQ ID NO: 3 (5'-UGAAGUUAUCAUCUGACAGGAUUUC-3'), and siRNA consisting of the combination of sense RNA containing a contiguous base sequence of 15 to 24 bases in the base sequence represented by SEQ ID NO: 2 and antisense RNA containing a base sequence complementary to the aforementioned sense RNA; and having an RNA interference effect on NEK10 variant gene. In addition, the siRNA may also be siRNA consisting of the combination of sense RNA containing a base sequence in which one or a plurality of bases in a contiguous base sequence of 15 or more bases in the base sequence represented by SEQ ID NO: 2 is substituted, added or deleted, and antisense RNA containing a base sequence complementary to the aforementioned sense RNA, and having an RNA interference effect on NEK10 variant gene. In addition, the siRNA may also be siRNA in which one or a plurality of the bases in these siRNA is modified while also having an RNA interference effect on NEK10 variant gene.

Specific examples of siRNA targeting the entire length, or a partial length thereof, of the base sequence represented by SEQ ID NO: 4 in NEK10 variant mRNA include: siRNA consisting of the combination of sense RNA consisting of the base sequence represented by SEQ ID NO: 4 (5'-UCUGCCUUGUUUGUUCACCACUAUU-3') and antisense RNA consisting of the base sequence represented by SEQ ID NO: 5 (5'-AAUAGUGGUGAACAAACAAGGCAGA-3'), and siRNA consisting of the combination of sense RNA containing a contiguous base sequence of 15 to 24 bases in the base sequence represented by SEQ ID NO: 4 and antisense RNA containing a base sequence complementary to the aforementioned sense RNA; and having an RNA interference effect on NEK10 variant gene. In addition, the siRNA may also be siRNA consisting of the combination of sense RNA containing a base sequence in which one or a plurality of bases in a contiguous base sequence of 15 or more bases in the base sequence represented by SEQ ID NO: 4 is substituted, added or deleted, and antisense RNA containing a base sequence complementary to the aforementioned sense RNA; and having an RNA interference effect on NEK10 variant gene. In addition, the siRNA may also be siRNA in which one or a plurality of the bases in these siRNA is modified while also having an RNA interference effect on NEK10 variant gene.

[Precursor of RNAi Nucleic Acid Molecule]

In the method for inhibiting cell growth of the present invention, cells may be transfected directly with an RNAi nucleic acid molecule such as siRNA, or cells may be transfected with a precursor of the RNAi nucleic acid molecule and the RNAi nucleic acid molecule may be produced from the precursor by a reaction such as degradation within the cells. There are no particular limitations on the precursor of the RNAi nucleic acid molecule provided it is a precursor that ultimately produces an RNAi nucleic acid molecule such as siRNA in cells. Examples of siRNA precursors include comparatively long-stranded dsRNA and single-stranded RNA in which sense RNA and antisense RNA that constitute siRNA are coupled via a spacer. Although there are no particular limitations on the length of the spacer, it may be, for example, 3 to 23 bases in length. In addition, the spacer that couples the sense RNA and antisense RNA preferably forms a loop, and RNA sequences before and after the loop are preferably annealed to form a double strand (shRNA). Although there are no particular limitations on the length of the loop and stem in the shRNA, the length of the stemmay be, for example, 5 to 29 bases. In addition, an overhang consisting of several bases may or may not be present on the 5'-end and/or 3'-end. An RNAi nucleic acid molecule such as siRNA is produced by these precursors as a result of digesting with Dicer and the like within cells.

An example of a precursor used in the method for inhibiting cell growth of the present invention is shRNA having an RNA interference effect that targets a base sequence represented by SEQ ID NO: 2 or SEQ ID NO: 4 in mRNA of NEK10 variant gene. The precursor used in the present invention is preferably shRNA capable of producing in cells either siRNA targeting the entire length, or partial length, of the base sequence represented by SEQ ID NO: 2 in NEK10 variant mRNA, or siRNA targeting the entire length, or a partial length, of the base sequence represented by SEQ ID NO: 4 in NEK10 variant mRNA.

Examples of nucleic acid molecules that are precursors for producing siRNA targeting the entire sequence, or a partial sequence, of the base sequence represented by SEQ ID NO: 2 include shRNA containing a contiguous base sequence of 15 or more bases in the base sequence represented by SEQ ID NO: 2, or a base sequence in which one or a plurality of bases in that base sequence is modified, substituted, added or deleted; and a contiguous base sequence of 15 or more bases represented by SEQ ID NO: 3, or a base sequence in which one or a plurality of bases in that sequence is modified, substituted, added or deleted; and being capable of producing siRNA having an RNA interference effect on NEK10 variant gene from that shRNA in cells. Examples of nucleic acid molecules that are precursors for producing siRNA targeting the entire sequence, or a partial sequence, of the base sequence represented by SEQ ID NO: 4 include shRNA containing a contiguous base sequence of 15 or more bases in the base sequence represented by SEQ ID NO: 4, or a base sequence in which one or a plurality of bases in that base sequence is modified, substituted, added or deleted; and a contiguous base sequence of 15 or more bases represented by SEQ ID NO: 5, or a base sequence in which one or a plurality of bases in that sequence is modified, substituted, added or deleted, and being capable of producing siRNA having an RNA interference effect on NEK10 variant gene from that shRNA in cells.

An RNAi nucleic acid molecule such as siRNA or a nucleic acid molecule that is a precursor thereof can be suitably prepared by, for example, a chemical in vitro synthesis system, in vitro transcription method using phage RNA polymerase, or method in which long dsRNA that has been conjugated by transcribing using cloned cDNA as a template is cleaved by RNase III or Dicer. In addition, in the case of using an RNAi nucleic acid molecule containing a modified base, modification may be carried out on a synthesized nucleic acid molecule, or an RNAi nucleic acid molecule may be synthesized using the modified base.

[Expression Vector]

In the method for inhibiting cell growth of the present invention, an RNAi nucleic acid molecule and the like may be produced from an expression vector capable of expressing an RNAi nucleic acid molecule or precursor thereof in cells by transfecting the cells with the expression vector. Examples of vectors capable of expressing siRNA include expression vectors in which separate promoters are respectively coupled so as to separately express sense RNA and antisense RNA of siRNA, and expression vectors designed so that sense RNA and antisense RNA are separately transcribed from a single promoter by selective splicing and the like. Examples of vectors capable of expressing shRNA include expression vectors in which a single-stranded RNA that constitute shRNA is coupled downstream from a promoter.

These expression vectors can be easily fabricated by a person with ordinary skill in the art using ordinary genetic engineering techniques (T.R. Brummelkamp, et al., Science, 2002, Vol. 296, pp. 550-553; N.S. Lee, et al., Nat. Biotech., 2001, Vol. 19, pp. 500-505; M. Miyagishi and K. Taira, Nat. Biotech., 2002, Vol. 19, pp. 497-500; P.J. Paddison, et al., Proc. Natl. Acad. Sci. USA, 2002, Vol. 99, pp. 1443-1448; C.P. Paul, et al., Nat. Biotech., 2002, Vol. 19, p. 505-508; G. Sui, et al., Proc. Natl. Acad. Sci. USA, 2002, Vol. 99, pp. 5515-5520; G.M. Barton and R. Medzhitov, Proc. Natl. Acad. Sci. USA, 2002, Vol. 99, pp. 14943-14945; P.J. Paddison, et al., Genes Dev., 2002, Vol. 16, pp. 948-958). More specifically, DNA encoding a target RNA sequence can be constructed by suitably inserting various known expression vectors. RNA polymerase III promoter and the like can be used as promoter. More specifically, a promoter such as U6 promoter or H1 promoter can be used. Examples of known vectors that can be used include viral vectors such as retrovirus vector, adenovirus vector, adeno-associated viral vectors or minus-strand RNA viral vectors, and non-viral vectors such as plasmids.

[Cell Transfection]

There are no particular limitations on the method used to transfect cells with an RNAi nucleic acid molecule such as siRNA, precursor thereof, or expression vector thereof, and any known technique used when transfecting cells with nucleic acid molecules may be used. For example, cells may be transfected with an RNAi nucleic acid molecule and the like by injection, or may be allowed to be incorporated into cells by endocytosis and the like using a known gene transfection reagent as necessary. In addition, cells may be transfected with one type of RNAi nucleic acid molecule and the like or transfected with two or more types in combination.

In the method for inhibiting cell growth of the present invention, those cells targeted for inhibition of growth are cells in which NEK10 variant gene is expressed, or in other words, cells capable of expressing the transcription product of NEK10 variant gene in the form of mRNA or the translation product thereof in the form of protein (to be referred to as NEK10 variant-expressing cells). There are no particular limitations on the NEK10 variant-expressing cells, and they may be normal cells or cancer cells. In addition, there are no particular limitations on the organ from which they are derived. For example, there are no particular limitations on the type of cancer in the case of cancer cells. In addition, the cells may be of human origin or may be derived from an animal other than a human. The cells targeted for inhibition of growth may be cultured cells, cells collected from a living individual, or cells present in a living individual.

<Composition and Anticancer Agent for Inhibiting Expression of NEK10 Variant Gene>

When cells are transfected with a nucleic acid molecule such as siRNA having an RNA interference effect on NEK10 variant mRNA, a precursor of that siRNA (and particularly, shRNA), an expression vector of the aforementioned siRNA or an expression vector of the aforementioned precursor, as a result of RNA interference occurring in the cells and expression of NEK10 variant gene decreasing, growth of the cells is inhibited. In other words, these nucleic acid molecules are obtained in the form of active ingredients of a cell growth inhibitor, and are therefore substances that are useful as pharmaceuticals against diseases caused by hyperproliferation of cells such as tumor cells. Therefore, these nucleic acid molecules can be used, either directly or after suitably mixing with a pharmacologically acceptable compounding agent, as a composition (composition of present invention) or anticancer agent (anticancer agent of present invention) for inhibiting expression of NEK10 variant gene. Furthermore, the composition and cancer agent of the present invention may contain only one type of nucleic acid molecule such as siRNA or a plurality of types may be contained in combination.

The anticancer agent of the present invention having as an active ingredient thereof a nucleic acid molecule having an RNA interference effect on NEK10 variant mRNA is considered to be useful in the treatment of cancer that expresses NEK10 variant gene. NEK10 variant gene is expressed not only in breast cancer, but also at least in liver cancer, kidney cancer, prostate cancer and uterine cancer. Consequently, the anticancer agent of the present invention is expected to demonstrate a high level of antitumor effects in numerous types of cancer cells by affecting the cell growth thereof.

The composition and anticancer agent of the present invention demonstrate anticancer effects such as a cancer cell growth inhibitory effect, cancer cell death-inducing effect or sensitivity-enhancing effect with respect to anticancer agents and the like. In addition, these effects of the composition and anticancer agent of the present invention may be demonstrated transiently or permanently. In addition, the effects may be ultimately demonstrated after a fixed amount of time has elapsed following transfection with the composition or anticancer agent of the present invention.

Examples of compounding agents that may be added to the composition and anticancer agent of the present invention include pharmacologically acceptable additives such as carriers, excipients, disintegrating agents, binders, lubricants, fluidizers, coating agents, suspending agents, emulsifying agents, stabilizers, preservatives, correctives, flavoring agents, diluents or solubility assistants. In addition, the composition and anticancer agent of the present invention is safely administered orally or parenterally (including systemic administration and local administration) in a preparation form such as a powder, granules, tablet, caplet, capsule, injection, suppository or ointment. Although varying according to the preparation, the content of the active ingredient (RNAi nucleic acid molecule or nucleic acid molecule in the form of a precursor thereof) in the composition and anticancer agent of the present invention is normally preferably 0.1% by weight to 100% by weight. Although varying according to such factors as the administration route, patient age or actual symptoms to be prevented or treated, the dosage is, for example, 0.01 mg to 2000 mg per day, and preferably 0.1 mg to 1000 mg per day, as the amount of active ingredient in the case of orally administering to an adult, and the dosage can be administered once per day or divided among several administrations.

<Method for Screening Anticancer Agents>

As was previously described, growth of cancer cells is inhibited and antitumor effects are demonstrated as a result of inhibiting the function of NEK10 variant protein in cancer cells by decreasing expression of NEK10 variant gene. Consequently, substances having an effect that inhibits expression of NEK10 variant gene or substances having an effect that inhibits activity of NEK10 variant protein have a high possibility of being useful as active ingredients of cancer therapeutic drugs. Thus, novel anticancer agents can be screened by using the inhibitory effect on expression of NEK10 variant gene or the inhibitory effect on activity of NEK10 variant protein as an indicator.

Examples of candidate substances of anticancer agents include nucleic acids, peptides, proteins, organic compounds (including low molecular weight compounds and high molecular weight compounds) and inorganic compounds. The screening method of the present invention can be carried out on samples containing these candidate substances (to be referred to as test substances). Samples containing test substances include cell extracts, expression products of gene libraries, microbial culture supernatants, fungal components and the like.

More specifically, in the case of searching among test substances by using the inhibitory effect on expression of NEK10 variant gene as an indicator, NEK10 variant gene-expressing cells are cultured in the presence or absence of a test substance followed by comparing expression levels of NEK10 variant mRNA or NEK10 variant protein under both conditions.

The cells used in screening are only required to be NEK10 variant-expressing cells. Although human-derived NEK10 variant-expressing cells are preferably used in the case of screening effective anticancer agents in humans, NEK10 variant-expressing cells derived from an animal other than a human may also be used provided the cells are derived from a species in which a base sequence indicating homology with human NEK10 variant mRNA is present. For example, since a base sequence indicating homology with human NEK10 variant mRNA (NCBI accession no.: NM_001195119.1) is present in mouse transcription products, mouse-derived NEK10 variant-expressing cells may also be used. Furthermore, the range of cells used in screening includes tissue that is a collection of cells. In addition, transformed cells prepared in a state that enables production of NEK10 variant protein by transfecting with an expression vector having cDNA of human NEK10 variant gene in accordance with established methods can also be used as NEK10 variant-expressing cells.

In the aforementioned screening method, contact between a test substance and NEK10 variant-expressing cells can be carried out by, for example, culturing the NEK10 variant-expressing cells in a state in which the test substance is added to a culture solution of the NEK10 variant-expressing cells. In addition, although there are no particular limitations thereon, culturing conditions (such as temperature, pH and medium composition) that enable the cells to express NEK10 variant mRNA or protein without destroying the cells are preferably selected for the conditions under which a test substance contacts NEK10 variant-expressing cells.

Candidate substances can be screened by contacting a test substance with NEK10 variant-expressing cells under the aforementioned conditions, for example, and searching for those substances that cause a decrease in the expression level of NEK10 variant mRNA or protein. More specifically, in the case of having cultured NEK10 variant-expressing cells in the presence of a test substance, a test substance is selected for which the expression level of NEK10 variant mRNA or protein is less than the expression level of NEK10 variant mRNA or protein in the absence of the test substance under the same conditions.

The expression level of NEK10 variant mRNA or protein can be measured by a measurement method using northern blotting or a DNA array that uses an oligonucleotide probe having a sequence complementary to the base sequence of NEK10 variant mRNA, or by RT-PCR or real-time PCR using partial base sequences present in NEK10 variant mRNA as primers.

The expression level of NEK10 variant protein can be measured by, for example, a known method that uses antibody to NEK10 variant protein. Examples of measurement methods that use antibody include western blotting, immunoprecipitation and ELISA.

NEK10 variant is predicted to have kinase activity on the basis of its structure. Thus, anticancer agents can be screened by using as an indicator an inhibitory effect on the kinase activity of NEK10 variant protein. More specifically, the kinase activity of NEK10 variant protein is compared in the presence and absence of a test substance using purified NEK10 variant protein or an extract of cells expressing NEK10 variant protein.

A recombinant protein produced by gene recombination technology can be used as NEK10 variant protein during measurement of kinase activity. Recombinant protein can be produced in accordance with ordinary methods using cDNA of NEK10 variant gene and a known expression system. Examples of expression systems include expression systems using E. coli, yeast, insect cells or mammalian cells as host cells as well as cell-free expression systems. For example, an extract of host cells following forced expression of NEK10 variant protein may be used directly in measurement of kinase activity, or recombinant protein purified from the cell extract may be used. In addition, an extract of cells inherently expressing NEK10 variant protein, or NEK10 variant protein purified from that extract, can also be used to measure kinase activity.

Various types of proteins commonly used as kinase substrates can be used for the substrate used during measurement of kinase activity. Specific examples thereof include myelin basic protein (MBP) and histone. The protein used as substrate may be a recombinant protein, may be artificially synthesized by peptide synthesis and the like, or may be an intrinsic protein present in cells. In the case of using a recombinant protein or a protein intrinsically present in cells, a cell extract or protein purified from that extract may be used to measure kinase activity.

In the aforementioned screening method, a test substance and NEK10 variant-expressing cells are contacted in the environment used for measurement of kinase activity. Conditions such as the temperature, pH or salt concentration during measurement of kinase activity consist of those conditions under which the protein serving as substrate is phosphorylated by NEK10 variant protein, and there are no particular limitations thereon.

Selection of a test substance can be carried out by comparing the amount of substrate protein phosphorylated by NEK10 variant protein in the presence of the test substance under the aforementioned conditions and the amount of substrate phosphorylated by NEK10 variant protein in the absence of the test substance. More specifically, a test substance is selected for which the phosphorylated amount of substrate protein in the presence of the test substance is less than the phosphorylated amount of substrate protein in the absence of the test substance under the same conditions.

A substance selected according to the method for screening anticancer agents of the present invention has an effect that either decreases production of NEK10 variant protein or decreases the kinase activity of NEK10 variant protein by inhibiting expression of NEK10 variant gene in cells, and is considered to be useful in the treatment of cancer. In addition, derivatives having superior efficacy and safety can also be obtained by producing various derivatives of test substances selected according to this screening method and carrying out further screening thereon.

## Examples

Although the following provides a more detailed explanation of the present invention through examples thereof, these examples are only intended to be exemplary, and the present invention is not limited thereto. Furthermore, commercially available reagents mentioned in the examples were used in accordance with the manufacturer's instructions unless specifically indicated otherwise.

### [Reference Example 1]

The present reference example was carried out in order to confirm expression of NEK10 variant mRNA in various cancer cell lines. Namely, the relative expression levels of NEK10 variant mRNA in the various cancer cell lines listed in Table 1 were measured by real-time PCR using SYBR™ Green.

Total RNA was purified from each cancer cell line using the RNAeasy Kit (Qiagen). cDNA was prepared using 400 ng of purified RNA by using the reverse transcriptase Superscript III (Invitrogen) in accordance with the instructions. Namely, RNA was subjected to denaturation treatment for 5 minutes at 65°C followed by rapidly cooling at 4°C and subsequently allowing to react for 30 minutes at 55°C and then for 15 minutes at 75°C to synthesize cDNA.

Using 1 μL of the resulting cDNA as template, real-time PCR was carried out using a primer for amplifying cDNA synthesized from NEK10 variant mRNA, a primer for amplifying cDNA synthesized from GAPDH, and Brilliant SYBR™ Green Master Mix (Stratagene) in accordance with the instructions. Amplification of GAPDH was carried out as a control for the reaction system. Namely, after heat-denaturing for 10 minutes at 95°C, 40 cycles of PCR were carried out with one cycle consisting of 30 seconds at 95°C, 60 seconds at 55°C and 60 seconds at 72°C. The MX3000 (Stratagene) system was used for real-time PCR. The primers used to amplify cDNA synthesized from NEK10 variant mRNA consisted of a forward primer composed of the base sequence of SEQ ID NO: 6 (5'-GCACACAAAGGTATTTTATGG-3') and a reverse primer composed of the base sequence of SEQ ID NO: 7 (5'-CTACTCAAACTTGCCTTCTCA-3'). In addition, the primers used to amplify cDNA synthesized from GAPDH mRNA consisted of a forward primer composed of the base sequence of SEQ ID NO: 8 (5'-TCTGCTCCTCCTGTTCGACAGT-3') and a reverse primer composed of the base sequence of SEQ ID NO: 9 (5'-ACCAAATCCGTTGACTCCGAC-3'). MX Pro software (Stratagene) was used to determine Ct values. Ct value refers to the threshold number of cycles at which amplification of a target gene by a PCR reaction occurs exponentially (cycle threshold).

The ΔCt values (relative Ct value in the case of having compared with a calibrator) of NEK10 variant mRNA of various cancer cell lines were determined from the resulting Ct values using Equation (1) indicated below.

Equation (1):

ΔCt (NEK10 gene variant) value = Ct value of NEK10 variant mRNA - Ct value of GAPDH mRNA

The relative expression levels of NEK10 variant mRNA in each cancer cell line were determined according to Equation (2) based on a value of 100 for the expression level of NEK10 variant mRNA in HeLaS3 cells by using the ΔCt values of NEK10 variant mRNA in each of the cancer cell lines obtained from Equation (1).

Equation (2):

Relative expression level of NEK10 variant mRNA in each cancer cell line = (ΔCt value of NEK10 variant mRNA in each cancer cell line/ΔCt value of NEK10 variant mRNA in HeLaS3 cells) × 100

The relative expression levels of NEK10 variant mRNA in each cancer cell line based on a value of 100 for the expression level of NEK10 variant mRNA in HeLaS3 cells obtained using the aforementioned Equation (2) are shown in Table 1. Expression of NEK10 variant mRNA was observed in breast cancer, liver cancer, kidney cancer, prostate cancer and uterine cancer cells. Thus, NEK10 variant is predicted to play an important role not only in breast cancer cells, but in other cancer cells as well.

[Table 1]

| Cell Line | Cancer | NEK10 Variant mRNA (% of HeLaS3) |
|---|---|---|
| MDA-MB-157 | Breast cancer | 965 |
| MDA-MB-231 | Breast cancer | 156 |
| MDA-MB-468 | Breast cancer | 227 |
| ZR-75-30 | Breast cancer | 121 |
| HepG2 | Liver cancer | 217 |
| HLE | Liver cancer | 435 |
| A498 | Kidney cancer | 166 |
| Caki-1 | Kidney cancer | 112 |
| PC3 | Prostate cancer | 108 |
| HeLaS3 | Uterine cancer | 100 |

[Example 1]

The present example was carried out in order to confirm that siRNA having an RNA interference effect on NEK10 variant mRNA inhibits expression of NEK10 variant mRNA and inhibits growth of cancer cells.

<Confirmation of Expression Inhibitory Effect on NEK10 Variant mRNA>

After carrying out siRNA treatment by transfecting cells with siRNA consisting of the combination of sense RNA consisting of the base sequence represented by SEQ ID N0: 2 and antisense RNA consisting of the base sequence represented by SEQ ID NO: 3 (to be referred to as NEK10 siRNA #1) and siRNA consisting of the combination of sense RNA consisting of the base sequence represented by SEQ ID NO: 4 and antisense RNA consisting of the base sequence represented by SEQ ID NO: 5 (to be referred to as NEK10 siRNA #2), NEK10 variant mRNA was measured by real-time PCR using SYBR™ Green to study the knockdown effects on NEK10 variant mRNA.

Breast cancer cell line MDA-MB-231, which was confirmed to express NEK10 variant mRNA in Example 1, was transfected with control siRNA, NEK10 siRNA #1 or NEK10 siRNA #2, and NEK10 variant mRNA following siRNA treatment was measured by real-time PCR using SYBR™ Green to study the knockdown effects on NEK10 variant mRNA of each siRNA. The control siRNA was purchased from Invitrogen, and siRNA synthesized by Invitrogen was used for NEK10 siRNA #1 and NEK10 siRNA #2.

MDA-MB-231 cells were disseminated into a 12-well plate at 10,000 cells per well, and transfected with siRNA 24 hours later using Lipofectamine RNAiMAX (Invitrogen) in accordance with the manufacturer's instructions. Namely, a solution obtained by dissolving 1 μL of siRNA (20 nM) in 50 μL of OptiMEM (Invitrogen) was mixed with a solution obtained by dissolving 1 μL of Lipofectamine RNAiMAX in 50 μL of OptiMEM and allowed to stand undisturbed for 5 minutes at room temperature, followed by further allowing to stand undisturbed for 20 minutes at room temperature. After standing, the aforementioned mixture was added to the cells, and after culturing for 3 hours at 37°C, the medium was replaced followed by additionally culturing for 72 hours. Subsequently, the medium was removed and RNA was purified from the cells in each well using the RNAeasy Kit (Qiagen).

Using 500 ng of the purified RNA, cDNA was prepared in the same manner as Reference Example 1 using the reverse transcriptase Superscript III (Invitrogen). Ct values were measured in the same manner as Reference Example 1 using 1 μL of the resulting cDNA as template. The reagents, primers and equipment used were the same as those used in Reference Example 1.

The experiment was conducted in triplicate, the average value of the triplicate Ct values was substituted into the following Equation (3) to determine the ΔCt values of each siRNA treatment group. In addition, the experiment was repeated three times.

Equation (3)-1:

ΔCt (control) value = Ct value of NEK10 variant mRNA of siRNA treatment group - Ct value of GAPDH mRNA of control siRNA treatment group

Equation (3)-2:

ΔCt (sample) value = Ct value of NEK10 variant mRNA of NEK10 siRNA treatment group - Ct value of GAPDH mRNA of NEK10 siRNA treatment group

The expression level of NEK10 variant mRNA attributable to each siRNA treatment in each experiment was determined according to the following Equation (4) as the relative value based on a value of 100 for the first control siRNA treatment group using the ΔCt values of each siRNA treatment group obtained with the aforementioned Equation (3). However, in Equation (4), the amount of NEK10 variant mRNA is a percentage (%) relative to the control siRNA treatment group.

Equation (4):

$$\text{Amount of NEK10 variant mRNA} = 2^{(\Delta\text{Ct (control) value} - \Delta\text{Ct (sample) value})} \times 100$$

The calculation results are shown in FIG. 1. In FIG. 1, the relative expression levels of NEK10 variant mRNA of each siRNA treatment group based on a value of 100 for the expression level of NEK10 variant mRNA during control siRNA treatment are plotted on the vertical axis. Each siRNA treatment group is plotted on the horizontal axis.

The percentages of NEK10 mRNA attributable to control siRNA or NEK10 siRNA treatment versus the control (%) in breast cancer cell line MDA-MB-231 were 107±6.2 for the control siRNA treatment group, 11±0.5 for the NEK10 siRNA #1 treatment group, and 86±7.4 for the NEK10 siRNA #2 treatment group. As a result of testing the control siRNA treatment group and the NEK10 siRNA treatment groups using Dunnett's test, the NEK10 siRNA #1 group significantly inhibited NEK10 variant mRNA in comparison with the control siRNA treatment group (***, $p<0.001$). Similarly, the NEK10 siRNA #2 treatment group also significantly inhibited expression of NEK10 variant mRNA in comparison with the control siRNA treatment group (**, $p<0.01$). Namely, both NEK10 siRNA #1 and NEK10 siRNA #2 were clearly demonstrated to inhibit expression of NEK10 variant mRNA in breast cancer cells.

<Growth Inhibitory Effect of NEK10 siRNA on Cancer Cells>

NEK10 siRNA #1 and NEK10 siRNA #2 were confirmed to have growth inhibitory effects on cancer cells. Namely, a study was conducted by methylene blue assay to determine whether cell growth is inhibited by knocking down NEK10 variant gene by transfecting the cells with NEK10 siRNA #1 and NEK10 siRNA #2.

MDA-MB-231 cells were transfected with each siRNA in the same manner as previously described (Confirmation of Expression Inhibitory Effect on NEK10 Variant mRNA) followed by culturing for 72 hours after transfection. After culturing, the medium was removed followed by the addition of 1000 μL of methanol and allowing to stand for 2 minutes at room temperature to fix the cells. After removing the methanol, 1000 μL of dye solution (0.05% methylene blue solution) were added followed by dyeing for 30 minutes. After washing three times with 4 mL of distilled water, 2 mL of a 3% HCl solution were added followed by measurement of the absorbance of methylene blue at 660 nm using a microplate reader (BioRad).

The experiment was conducted in triplicate and the same experiment was repeated three times. The average triplicate value of the control siRNA treatment group of the first experiment was used as a control, the cell growth rates (%) were calculated according to the following Equation (5) using the average triplicate values of the siRNA treatment group of each experiment.

Equation (5):

Cell growth rate (%) = (absorbance at 660 nm of control siRNA treatment group or NEK10 siRNA treatment group of each experiment/absorbance at 660 nm of control siRNA treatment group of first experiment) × 100

The calculation results are shown in FIG. 2. The cell growth rates of each siRNA treatment group based on a value of 100 for cell growth during control siRNA treatment are plotted on the vertical axis. Each siRNA treatment group is plotted on the horizontal axis.

The cell growth rates(%) of the MDA-MB-231 breast cancer cells attributable to each siRNA treatment were 88±18 for the control siRNA treatment group, 34±14 for the NEK10 siRNA #1 treatment group, and 58±17 for the NEK10 siRNA #2 treatment group. As a result of testing the control siRNA treatment group and the NEK10 siRNA treatment groups using Dunnett's test, the NEK10 siRNA #1 group significantly inhibited the growth of MDA-MB-231 cells in comparison with the control siRNA treatment group (*, $p<0.05$).

In addition, although statistical significance was unable to be confirmed for the NEK10 siRNA #2 group, it inhibited the growth of MDA-MB-231 cells to a greater degree than the control siRNA treatment group. Namely, although there are differences in the degree of their effects, both NEK10 siRNA #1 and NEK10 siRNA #2 were clearly determined to have growth inhibitory effects on breast cancer cells.

In addition, NEK10 siRNA #1, which demonstrated greater expression inhibitory effects on NEK10 variant mRNA, also demonstrated greater cell growth inhibitory effects than NEK10 siRNA #2. As a result, it was suggested that greater cell growth inhibitory effects are obtained by using siRNA having greater expression inhibitory effects on NEK10 variant mRNA, and that in order to obtain adequate cell growth inhibitory effects, it is preferable to use siRNA capable of obtaining an expression level of NEK10 variant mRNA of 86 or lower versus the control (%). In addition, since NEK10 siRNA #1 and NEK10 siRNA #2 target different sequences of NEK10 variant mRNA, concomitant use of NEK10 siRNA #1 and NEK10 siRNA #2 is expected to allow the obtaining of greater cell growth inhibitory effects (antitumor effects) than in the case of using either siRNA alone.

## INDUSTRIAL APPLICABILITY

Since the method for inhibiting cell growth and nucleic acid molecule of the present invention are able to inhibit growth of various cells, and particularly cancer cells, that express NEK10 variant gene, they can be used in fields such as the treatment of cancer and the production of anticancer agents.

## Claims

**1.** A method for inhibiting cell growth, comprising:

an expression decreasing step for decreasing expression of NEK10 variant gene, and/or
an activity decreasing step for decreasing activity of NEK10 variant protein
in cells.

**2.** The method for inhibiting cell growth according to claim 1, wherein the expression decreasing step is a step for transfecting cells with at least one type selected from the group consisting of: a nucleic acid molecule that inhibits expression of NEK10 variant gene by RNA interference, a precursor of the nucleic acid molecule, and an expression vector capable of expressing the nucleic acid molecule or the precursor.

**3.** The method for inhibiting cell growth according to claim 2, wherein the nucleic acid molecule is siRNA having an RNA interference effect that targets a base sequence in mRNA of NEK10 variant gene represented by SEQ ID NO: 2 or SEQ ID NO: 4, and
the precursor is shRNA having an RNA interference effect that targets a base sequence in mRNA of NEK10 variant gene represented by SEQ ID NO: 2 or SEQ ID NO: 4.

**4.** The method for inhibiting cell growth according to claim 2, wherein the nucleic acid molecule is selected from the group consisting of:

(a) siRNA consisting of the combination of sense RNA consisting of the base sequence represented by SEQ ID NO: 2, and antisense RNA consisting of the base sequence represented by SEQ ID NO: 3,
(b) siRNA consisting of the combination of sense RNA consisting of the base sequence represented by SEQ ID NO: 4, and antisense RNA consisting of the base sequence represented by SEQ ID NO: 5,
(c) siRNA consisting of the combination of sense RNA containing a contiguous base sequence of 15 to 24 bases in the base sequence represented by SEQ ID NO: 2, and antisense RNA containing a base sequence complementary to the sense RNA; and having an RNA interference effect on NEK10 variant gene,
(d) siRNA consisting of the combination of sense RNA containing a contiguous base sequence of 15 to 24 bases in the base sequence represented by SEQ ID NO: 4, and antisense RNA containing a base sequence complementary to the sense RNA; and having an RNA interference effect on NEK10 variant gene,
(e) siRNA consisting of the combination of sense RNA containing a base sequence in which one or a plurality of the bases in a contiguous base sequence of 15 or more bases in the base sequence represented by SEQ ID NO: 2 is substituted, added or deleted, and antisense RNA containing a base sequence complementary to the sense RNA; and having an RNA interference effect on NEK10 variant gene,
(f) siRNA consisting of the combination of sense RNA containing a base sequence in which one or a plurality of the bases in a contiguous base sequence of 15 or more bases in the base sequence represented by SEQ ID NO: 4 is substituted, added or deleted, and antisense RNA containing a base sequence complementary to the sense RNA; and having an RNA interference effect on NEK10 variant gene, and
(g) siRNA in which one or a plurality of bases in the siRNA described in any of (a) to (f) is modified, and having an RNA interference effect on NEK10 variant gene.

**5.** The method for inhibiting cell growth according to claim 2, wherein the precursor produces in cells any of:

(a) siRNA consisting of the combination of sense RNA consisting of the base sequence represented by SEQ ID NO: 2 and antisense RNA consisting of the base sequence represented by SEQ ID NO: 3,
(b) siRNA consisting of the combination of sense RNA consisting of the base sequence represented by SEQ ID NO: 4 and antisense RNA consisting of the base sequence represented by SEQ ID NO: 5,
(c) siRNA consisting of the combination of sense RNA containing a contiguous base sequence of 15 to 24 bases in the base sequence represented by SEQ ID NO: 2 and antisense RNA containing a base sequence complementary to the sense RNA; and having an RNA interference effect on NEK10 variant gene,
(d) siRNA consisting of the combination of sense RNA containing a contiguous base sequence of 15 to 24 bases in the base sequence represented by SEQ ID NO: 4 and antisense RNA containing a base sequence complementary to the sense RNA; and having an RNA interference effect on NEK10 variant gene,
(e) siRNA consisting of the combination of sense RNA containing a base sequence in which one or a plurality of the bases in a contiguous base sequence of 15 or more bases in the base sequence represented by SEQ ID NO: 2 is substituted, added or deleted, and antisense RNA containing a base sequence complementary to the sense RNA; and having an RNA interference effect on NEK10 variant gene,
(f) siRNA consisting of the combination of sense RNA containing a base sequence in which one or a plurality of the bases in a contiguous base sequence of 15 or more bases in the base sequence represented by SEQ ID NO: 4 is substituted, added or deleted, and antisense RNA containing a base sequence complementary to the sense RNA; and having an RNA interference effect on NEK10 variant gene, or
(g) siRNA in which one or a plurality of bases in the siRNA described in any of (a) to (f) is modified, and having an RNA interference effect on NEK10 variant gene.

**6.** The method for inhibiting cell growth according to claim 2, wherein the precursor is:

(p) shRNA containing a contiguous base sequence of 15 or more bases in the base sequence represented by SEQ ID NO: 2 or a base sequence in which one or a plurality of bases in that base sequence is modified, substituted, added or deleted, and a contiguous base sequence of 15 or more bases in the base sequence represented by SEQ ID NO: 3 or a base sequence in which one or a plurality of bases in that base sequence is modified, substituted, added or deleted,
or
(q) shRNA containing a contiguous base sequence of 15 or more bases in the base sequence represented by SEQ ID NO: 4 or a base sequence in which one or a plurality of bases in that base sequence is modified, substituted, added or deleted, and a contiguous base sequence of 15 or more bases in the base sequence represented by SEQ ID NO: 5 or a base sequence in which one or a plurality of bases in that base sequence is modified, substituted, added or deleted;
and,
siRNA, having an RNA interference effect on NEK10 variant gene, is produced in cells from the shRNA of (p) and the shRNA of (q) .

**7.** The method for inhibiting cell growth according to claim 1, wherein the cells are cancer cells.

**8.** A nucleic acid molecule that is:

(a) siRNA consisting of the combination of sense RNA consisting of the base sequence represented by SEQ ID NO: 2 and antisense RNA consisting of the base sequence represented by SEQ ID NO: 3,
(b) siRNA consisting of the combination of sense RNA consisting of the base sequence represented by SEQ ID NO: 4 and antisense RNA consisting of the base sequence represented by SEQ ID NO: 5,
(c) siRNA consisting of the combination of sense RNA containing a contiguous base sequence of 15 to 24 bases in the base sequence represented by SEQ ID NO: 2, and antisense RNA containing a base sequence complementary to the sense RNA; and having an RNA interference effect on NEK10 variant gene,
(d) siRNA consisting of the combination of sense RNA containing a contiguous base sequence of 15 to 24 bases in the base sequence represented by SEQ ID NO: 4, and antisense RNA containing a base sequence complementary to the sense RNA; and having an RNA interference effect on NEK10 variant gene,
(e) siRNA consisting of the combination of sense RNA containing a base sequence in which one or a plurality of the bases in a contiguous base sequence of 15 or more bases in the base sequence represented by SEQ ID NO: 2 is substituted, added or deleted, and antisense RNA containing a base sequence complementary to the sense RNA; and having an RNA interference effect on NEK10 variant gene,
(f) siRNA consisting of the combination of sense RNA containing a base sequence in which one or a plurality of the bases in a contiguous base sequence of 15 or more bases in the base sequence represented by SEQ ID NO: 4 is substituted, added or deleted, and antisense RNA containing a base sequence complementary to the sense RNA; and having an RNA interference effect on NEK10 variant gene, or
(g) siRNA in which one or a plurality of bases in the siRNA described in any of (a) to (f) is modified, and having an RNA interference effect on NEK10 variant gene.

**9.** A nucleic acid molecule that is:

(p) shRNA containing a contiguous base sequence of 15 or more bases in the base sequence represented by SEQ ID NO: 2 or a base sequence in which one or a plurality of bases in that base sequence is modified, substituted, added or deleted, and a contiguous base sequence of 15 or more bases in the base sequence represented by SEQ ID NO: 3 or a base sequence in which one or a plurality of bases in that base sequence is modified, substituted, added or deleted,
or
(q) shRNA containing a contiguous base sequence of 15 or more bases in the base sequence represented by SEQ ID NO: 4 or a base sequence in which one or a plurality of bases in that base sequence is modified, substituted, added or deleted, and a contiguous base sequence of 15 or more bases in the base sequence represented by SEQ ID NO: 5 or a base sequence in which one or a plurality of bases in that base sequence is modified, substituted, added or deleted;
and,
is a precursor for producing siRNA having an RNA interference effect on NEK10 variant gene in cells.

**10.** An expression vector containing the nucleic acid according to claim 8 or 9, which is capable of expressing that nucleic acid.

**11.** A composition for inhibiting expression of NEK10 variant gene, comprising one or more types selected from the group consisting of:

the nucleic acid molecule according to claim 8,
the nucleic acid molecule according to claim 9, and
the expression vector according to claim 10.

**12.** An anticancer agent containing as an active ingredient thereof one or more types selected from the group consisting of:

the nucleic acid molecule according to claim 8,
the nucleic acid molecule according to claim 9, and
the expression vector according to claim 10.

**13.** A method for screening anticancer agents using an inhibitory effect on expression of NEK10 variant gene or an inhibitory effect on activity of NEK10 variant protein as an indicator.

**14.** The method for screening anticancer agents according to claim 13, comprising:

a step for culturing NEK10 variant-expressing cells respectively in the presence and absence of a candidate substance for an inhibitory effect on expression of NEK10 variant gene or an inhibitory effect on activity of NEK10 variant protein, and
a step for measuring the expression level of NEK10 variant mRNA in cells or the amount of activity of NEK10 variant protein, and comparing the expression levels or amounts of activity in the presence and absence of the candidate substance.

*FIG. 1*

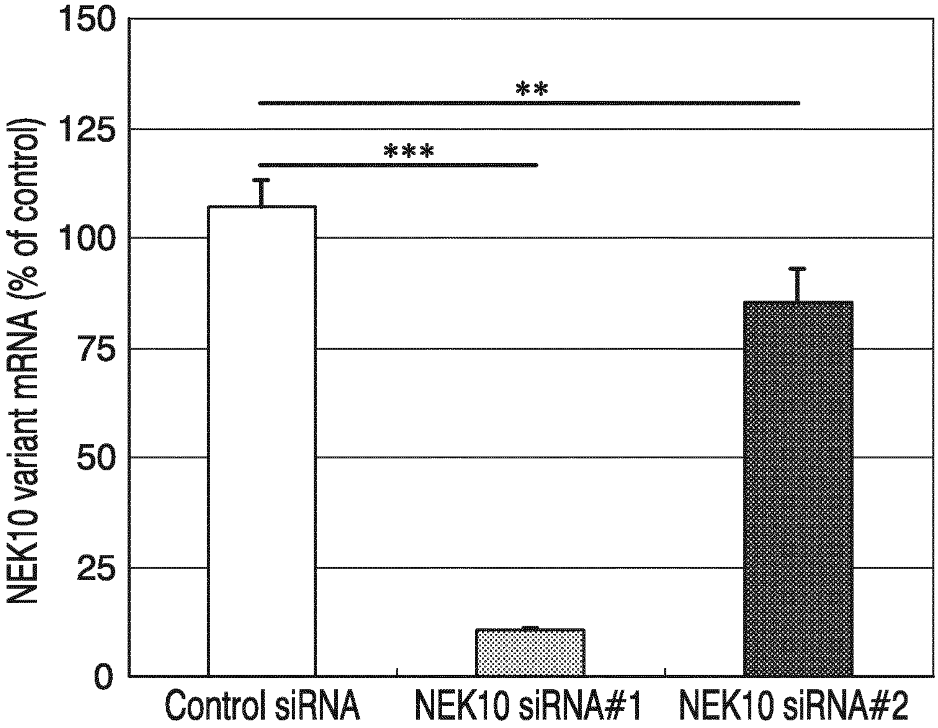
150
125
100
75
50
25
0
NEK10 variant mRNA (% of control)
**
***
Control siRNA
NEK10 siRNA#1
NEK10 siRNA#2

*FIG. 2*

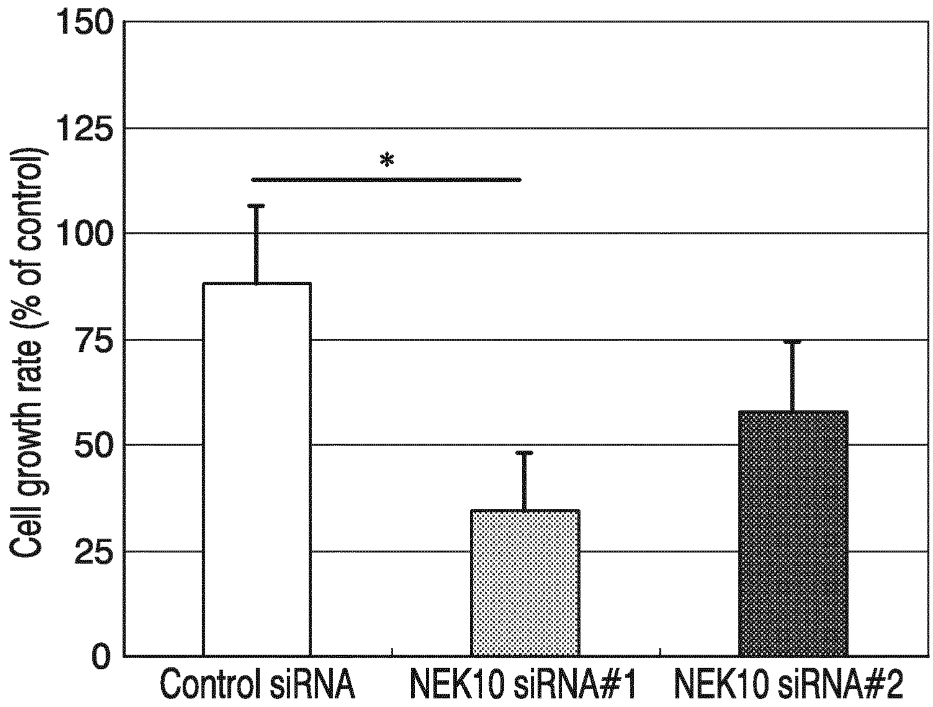
150
125
100
75
50
25
0
Cell growth rate (% of control)
*
Control siRNA
NEK10 siRNA#1
NEK10 siRNA#2

## INTERNATIONAL SEARCH REPORT

International application No.
PCT/JP2012/071868

A. CLASSIFICATION OF SUBJECT MATTER
*C12N15/00*(2006.01)i, *A61K31/713*(2006.01)i, *A61K48/00*(2006.01)i, *A61P35/00* (2006.01)i, *C12N15/09*(2006.01)i, *C12N15/113*(2010.01)i, *C12Q1/02*(2006.01)i, *C12Q1/68*(2006.01)i, *G01N33/15*(2006.01)i, *G01N33/50*(2006.01)i
According to International Patent Classification (IPC) or to both national classification and IPC

B. FIELDS SEARCHED
Minimum documentation searched (classification system followed by classification symbols)
C12N15/00, A61K31/713, A61K48/00, A61P35/00, C12N15/09, C12N15/113, C12Q1/02, C12Q1/68, G01N33/15, G01N33/50

Documentation searched other than minimum documentation to the extent that such documents are included in the fields searched

Electronic data base consulted during the international search (name of data base and, where practicable, search terms used)
CA/BIOSIS/MEDLINE(STN), JSTPlus/JMEDPlus/JST7580(JDreamII), GenBank/EMBL/DDBJ/GeneSeq, PubMed, CiNii, WPI

C. DOCUMENTS CONSIDERED TO BE RELEVANT

| Category* | Citation of document, with indication, where appropriate, of the relevant passages | Relevant to claim No. |
|---|---|---|
| Y | MONIZ, L. S. and STAMBOLIC, V., Nek10 mediates G2/M cell cycle arrest and MEK autoactivation in response to UV irradiation, MOLECULAR AND CELLULAR BIOLOGY, January 2011, Vol.31 No.1, pages 30-42. | 1-14 |
| Y | WO 2010/094734 A2 (BIOFOCUS DPI B.V.), 26 August 2010 (26.08.2010), entire text & EP 2398479 A2 & US 2012/004160 A1 | 1-14 |
| Y/A | Database GenBank, [online], 14 September 2006 (14.09.2006), Accession No. AK098832, [retrieval date 21 September 2012 (21.09.2012)], internet<URL:http://www.ncbi.nlm.nih.gov/nuccore/AK098832> | 1-7,12-14/ 8-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 September, 2012 (21.09.12) | 02 October, 2012 (02.10.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/071868

C (Continuation). DOCUMENTS CONSIDERED TO BE RELEVANT

| Category* | Citation of document, with indication, where appropriate, of the relevant passages | Relevant to claim No. |
|---|---|---|
| Y/A | LI, J. J. and LI, S. A., Mitotic kinases: the key to duplication, segregation, and cytokinesis errors, chromosomal instability, and oncogenesis, Pharmacology & Therapeutics, 2006, Vol.111, pages 974-984. | 1-7,12-14/ 8-11 |
| Y/A | BOWERS, A. J. and BOYLAN, J. F., Nek8, a NIMA family kinase member, is overexpressed in primary human breast tumors, GENE, 2004, Vol. 328, pages 135-142. | 1-7,12-14/ 8-11 |
| Y/A | BELHAM, C. et al., A mitotic cascade of NIMA family kinases, THE JOURNAL OF BIOLOGICAL CHEMISTRY, 2003, Vol.278 No.37, pages 34897-34909. | 1-7,12-14/ 8-11 |
| Y/A | FRY, A. M. et al., A centrosomal function for the human Nek2 protein kinase, a member of the NIMA family of cell cycle regulators, The EMBO Journal, 1998, Vol.17 No.2, pages 470-481. | 1-7,12-14/ 8-11 |
| Y/A | DAVIES, H. et al., Somatic mutations of the protein kinase gene family in human lung cancer, Cancer Res., 2005, Vol.65 No.17, pages 7591-7595. | 1-7,12-14/ 8-11 |
| Y/A | ANTONIOU, A. C. et al., Common breast cancer susceptibility alleles and the risk of breast cancer for BRCA1 and BRCA2 mutation carriers: implications for risk prediction, Cancer Res., 2010, Vol.70 No.23, pages 9742-9754. | 1-7,12-14/ 8-11 |
| Y/A | AHMED, S. et al., Newly discovered breast cancer susceptibility loci on 3p24 and 17q23.2, NATURE GENETICS, 2009, Vol.41 No.5, pages 585-590. | 1-7,12-14/ 8-11 |
| Y/A | MAVADDAT, N. et al., Familial relative risks for breast cancer by pathological subtype: a population-based cohort study, Breast Cancer RESEARCH, 2010, Vol.12 R10, pages 1-12. | 1-7,12-14/ 8-11 |
| Y/A | GORODNOVA, T. V. et al., Distribution of FGFR2, TNRC9, MAP3K1, LSP1, and 8q24 alleles in genetically enriched breast cancer patients versus elderly tumor-free women, Cancer Genetics and Cytogenetics, 2010, Vol.199, pages 69-72. | 1-7,12-14/ 8-11 |
| A | WO 2007/117038 A1 (Japanese Foundation for Cancer Research), 18 October 2007 (18.10.2007), entire text & EP 2018873 A1 & US 2009/202569 A1 | 1-14 |

Form PCT/ISA/210 (continuation of second sheet) (July 2009)

**INTERNATIONAL SEARCH REPORT**

International application No. PCT/JP2012/071868

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-282628 A (Daiichi Pharmaceutical Co., Ltd.), 01 November 2007 (01.11.2007), entire text (Family: none) | 1-14 |
| A | WO 2005/061704 A1 (Takeda Chemical Industries, Ltd.), 07 July 2005 (07.07.2005), entire text & EP 1712619 A1 & US 2007/275888 A1 | 1-14 |
| A | JP 2004-203745 A (Eisai Co., Ltd.), 22 July 2004 (22.07.2004), entire text (Family: none) | 1-14 |
| A | ZENG, M. et al., Identifying mRNAs bound by human RBMY protein in the testis, Journal of Reproduction and Development, March 2011, Vol. 57 No.1, pages 107-112. | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **M.J. O'CONNELL et al.** *Trends in Cell Biology,* 2003, vol. 13, 221-228
- **O'REGAN et al.** *Cell Division,* 2007, vol. 2, 25-36
- **L.S. MONIZ et al.** *Molecular and Cellular Biology,* 2011, vol. 31, 30-42
- **S. AHMED et al.** *Nature Genetics,* 2009, vol. 41, 585-590
- **A.C. ANTONIOU et al.** *Cancer Research,* 2010, vol. 70, 9742-9754
- **P. ANTON et al.** *Nature,* 2002, vol. 418, 38-39
- **L. DAVID et al.** *Nat. Genet.,* 2002, vol. 32, 107-108
- **T.R. BRUMMELKAMP et al.** *Science,* 2002, vol. 296, 550-553
- **N.S. LEE et al.** *Nat. Biotech.,* 2001, vol. 19, 500-505
- **M. MIYAGISHI ; K. TAIRA.** *Nat. Biotech.,* 2002, vol. 19, 497-500
- **P.J. PADDISON et al.** *Proc. Natl. Acad. Sci. USA,* 2002, vol. 99, 1443-1448
- **C.P. PAUL et al.** *Nat. Biotech.,* 2002, vol. 19, 505-508
- **G. SUI et al.** *Proc. Natl. Acad. Sci. USA,* 2002, vol. 99, 5515-5520
- **G.M. BARTON ; R. MEDZHITOV.** *Proc. Natl. Acad. Sci. USA,* 2002, vol. 99, 14943-14945
- **P.J. PADDISON et al.** *Genes Dev.,* 2002, vol. 16, 948-958

SEQUENCE LISTING
<110> Nippon Kayaku Co., Ltd.
<120> Method for inhibiting cell proliferation, nucleic acid molecule with RNA interference activity to NEK10 gene, and anticancer agent
<130> J70124A1
<160> 9

<210> 1
<211> 1215
<212> DNA
<213> Homo sapiens
<400> 1
```
atggagctga tagaaggagc cccgcttgga gagcatttca gttctttgaa ggaaaaacat        60
caccatttta ctgaagaaag actatggaaa atatttatac agctgtgctt agctcttcga       120
tacttacaca aggagaagag gattgtccat agagatctga caccaaacaa cattatgttg       180
ggggataagg acaaagtaac cgttactgac tttggcctgg caaagcaaaa acaagaaaac       240
agtaaactca cctctgtggt tggaacaatc ctgtattctt gccccgaggt actgaagagt       300
gagccgtatg gggagaaggc tgatgtctgg gcagtaggct gcatccttta tcagatggcg       360
actttgagtc ccccttcta cagcactaac atgctgtcct tggctacaaa aatagtggag        420
gcggtatatg aaccagtccc agaaggtatc tactctgaaa aagtaacaga caccatcagc       480
aggtgcctca ctcctgatgc ggaagctcgt ccagatattg tagaagtcag ttcgatgata       540
tcagatgtca tgatgaaata tttagacaac ttatctacat cccagttgtc cttggaaaag       600
aagctagaac gggaacgaag acgcacacaa aggtatttta tggaagccaa ccggaacacc       660
gtcacatgtc accatgagct ggctgttcta tctcacgaga cctttgagaa ggcaagtttg       720
agtagcagca gcagtggagc agccagcctg aaaagtgaac tttcagaaag cgcagacctg       780
ccccctgaag gcttccaggc ctcctatggt aaagacgaag acagggcctg tgacgaaatc       840
ctgtcagatg ataacttcaa cctggaaaat gctgagaaag atacatattc agaggtagat       900
gatgaattgg acatttcgga taactccagc agctccagtt caagcccccct gaaagaatct      960
acattcaaca ttttaaagag aagttttagt gcttcaggag gagaaagaca atcccaaaca      1020
agggacttca ctggaggaac aggatcaaga ccaagaccag ctttgctgcc tcttgacctg      1080
cttctgaaag tgccacccca catgctcagg gcccacatta aggaaataga ggctgagtta      1140
gtgacagggt ggcagtccca tagccttcct gctgtgattc ttcgaaatct caaagatcat      1200
ggtagtactt actag                                                      1215
```

<210> 2
<211> 25
<212> RNA
<213> Artificial Sequence
<220>
<223> Description of Artificial Sequence: sense RNA of siRNA for NEK10 variant
gene
<400> 2
```
gaaauccugu cagaugauaa cuuca                                            25
```

<210> 3
<211> 25
<212> RNA
<213> Artificial Sequence
<220>
<223> Description of Artificial Sequence: antisense RNA of siRNA for NEK10 variant gene
<400> 3
```
ugaaguuauc aucugacagg auuuc                                            25
```

<210> 4
<211> 25
<212> RNA
<213> Artificial Sequence
<220>
<223> Description of Artificial Sequence: sense RNA of siRNA for NEK10 variant
gene

<400> 4
ucugccuugu uuguucacca cuauu 25

<210> 5
<211> 25
<212> RNA
<213> Artificial Sequence
<220>
<223> Description of Artificial Sequence: antisense RNA of siRNA for NEK10 variant gene
<400> 5
aauaguggug aacaaacaag gcaga 25

<210> 6
<211> 21
<212> DNA
<213> Artificial Sequence
<220>
<223> Description of Artificial Sequence: Forward primer for NEK10 variant mRNA
<400> 6
gcacacaaag gtattttatg g 21

<210> 7
<211> 21
<212> DNA
<213> Artificial Sequence
<220>
<223> Description of Artificial Sequence: Reverse primer for NEK10 variant mRNA
<400> 7
ctactcaaac ttgccttctc a 21

<210> 8
<211> 22
<212> DNA
<213> Artificial Sequence
<220>
<223> Description of Artificial Sequence: Forward primer for GAPDH mRNA
<400> 8
tctgctcctc ctgttcgaca gt 22

<210> 9
<211> 21
<212> DNA
<213> Artificial Sequence
<220>
<223> Description of Artificial Sequence: Reverse primer for GAPDH mRNA
<400> 9
accaaatccg ttgactccga c 21